# EUROPEAN PATENT SPECIFICATION

(11) **EP 3 568 691 B1**
(45) Date of publication and mention of the grant of the patent: **30.09.2026**
(21) Application number: 17821920.0
(22) Date of filing: 21.12.2017
(51) Int. Cl.: G01N 25/48, G01N 33/48

(54) **OPTICAL THERMAL METHOD AND SYSTEM FOR DIAGNOSING PATHOLOGIES**
OPTISCHE THERMISCHE VERFAHREN UND VORRICHTUNG ZUR DIAGNOSE VON PATHOLOGIEN
PROCEDE ET DISPOSITIF OPTO-THERMIQUE POUR LE DIAGNOSE DE PATHOLOGIES

(30) Priority: 10.01.2017 EP 17150839
(43) Date of publication of application: 20.11.2019
(73) Proprietor: Universite d'Aix-Marseille (AMU), 13284 Marseille Cedex 07 (FR); Institut National de la Santé et de la Recherche Médicale, 75013 Paris (FR)
(72) Inventor: TSVETKOV, Philipp, 13005 Marseille (FR); DEVRED, François, 13005 Marseille (FR)
(74) Representative: Axe PI
(86) International application number: PCT/EP2017/084251
(87) International publication number: WO 2018/130405

(56) References cited:
- EP-A1- 2 942 103
- US-B1- 6 406 918
- ALEXANDER CRISPIN G ET AL: "Novel microscale approaches for easy, rapid determination of protein stability in academic and commercial settings", BIOCHIMICA ET BIOPHYSICA ACTA (BBA) - PROTEINS & PROTEOMICS, ELSEVIER, NETHERLANDS, vol. 1844, no. 12, 28 September 2014 (2014-09-28), pages 2241 - 2250, XP029091216, ISSN: 1570-9639, DOI: 10.1016/J.BBAPAP.2014.09.016
- GUILLERMO SENISTERRA ET AL: "Thermal Denaturation Assays in Chemical Biology", ASSAY AND DRUG DEVELOPMENT TECHNOLOGIES, vol. 10, no. 2, 1 April 2012 (2012-04-01), US, pages 128 - 136, XP055345827, ISSN: 1540-658X, DOI: 10.1089/adt.2011.0390
- CHAGOVETZ ALEXIS A ET AL: "Differential Scanning Calorimetry of Gliomas: A New Tool in Brain Cancer Diagnostics?", NEUROSURGERY,, vol. 73, no. 2, 1 August 2013 (2013-08-01), pages 289 - 295, XP009193514, ISSN: 0148-396X
- SPLINTER R ET AL: "Measuring human blood serum with chip based fast liquid differential scanning calorimetry", THERMOCHIMICA ACTA, ELSEVIER SCIENCE PUBLISHERS, AMSTERDAM, NL, vol. 639, 16 July 2016 (2016-07-16), pages 76 - 83, XP029694951, ISSN: 0040-6031, DOI: 10.1016/J.TCA.2016.07.013

## Description

### TECHNICAL FIELD

The invention relates to a method and to a system for diagnosing a pathology in a mammal in need thereof. The method according to the invention comprises the steps of using a biological sample from said mammal, the biological sample comprising a protein mixture, and obtaining a thermal denaturation profile of said biological sample using optical methods.

### BACKGROUND OF THE INVENTION

Proteins denaturation is a process in which the proteins lose their quaternary, tertiary and/or their secondary structure, which are present in their native state. This process generally involves the application of some external stresses or compounds such as strong acids or bases, concentrated inorganic salts, organic solvents, radiations or heat.

In most cases of the state of the art, proteins denaturation that is provided by heat involves (i) heating a protein solution at a constant rate, (ii) recording some physical or chemical properties of said protein solution, and (iii) determining the temperature according to which the protein denatures, also called denaturation temperature of the protein or the melting temperature (Tₘ). A known method that is implemented for protein thermal denaturation studies is named Differential Scanning Calorimetry (DSC). DSC allows to follow denaturation of the tertiary structure of a protein.

However, DSC is not the only method which allows to follow proteins denaturation. Optical methods such as, for example, fluorimetry or circular dichroism (CD) spectroscopy, are also used for studying proteins denaturation.

These optical methods have been however implemented to study denaturation of isolated and purified proteins.

DSC has been used to obtain denaturation profiles of biofluids containing a mixture of proteins and, in particular, of a protein mixture contained in blood plasma. Indeed, these biofluids contain thousands of components including salts, metal ions, proteins, and a number of different organic molecules.

Among them, proteins and nucleic acids having structure denature upon heating. Still, due to low concentration of nucleic acids, the overall biofluid denaturation profile is mostly due to proteins. Nevertheless, other components of biofluid, such as ions, and other organic molecules, that do not denature, could impact the denaturation profile of individual proteins, indirectly affecting biofluid denaturation profile.

The documents as follows are known from the state of the art: the patent document EP 2 942 103 A1, that discloses a system and a method for thermos-optical measurments in a droplet; the document ALEXANDER CRISPIN G ET AL: "Novel microscale approaches for easy, rapid determination of protein stability in academic and commercial settings", BIOCHIMICA ET BIOPHYSICA ACTA (BBA) - PROTEINS & - PROTEOMICS, ELSEVIER, NETHERLANDS, vol. 1844, no. 12, 28 September 2014, pages 2241-2250; and the document SPLINTER R ET AL: "Measuring human blood serum with chip based fast liquid differential scanning calorimetry", THERMOCHIMICA ACTA, ELSEVIER SCIENCE PUBLISHERS, AMSTERDAM, NL, vol. 639, 16 July 2016, pages 76-83.

The patent document US 2011/0301860 A1 discloses the use of DSC for diagnostic purpose. More specifically, it discloses a method for detecting inflammatory diseases such as for example celiac disease, systemic lupus erythematosus, idiopathic thrombocytopenic purpura using DSC.

However, DSC apparatus are mainly designed for scientific research and are not adapted to routine experiments with a large number of biological samples. In particular, from a practical point of view, a DSC apparatus records extremely low heat exchanges. Hence, the experimental cells have to be carefully isolated from external heat sources by adiabatic jackets. This caution makes it difficult to create DSC apparatus that allow to run multiple samples of small volume, routinely, in a same time. Multi Cell DSC apparatus do exist but their sensitivity is not sufficient to record denaturation profiles of biological samples. Additionally, the sample cells of existing microcalorimeters are not replaceable and have to be washed after each experiment in order to prevent interference with the next experiment. Last, but not least, DSC is a calorimetric method which is specific for studying only the denaturation of the tertiary structure of proteins.

Based on these observations, there remains a need for developing a diagnostic approach which is faster than the DSC diagnostic methods. Moreover, there still remains a need for developing a diagnostic method which allows to access to a more complex protein denaturation profiles. In other words, there remains a need for obtaining denaturation of quaternary, tertiary and/or secondary structures of proteins. This kind of experiments would allow to diagnose a larger panel of diseases.

### SUMMARY OF THE INVENTION

In view of the above, the invention proposes to develop a quite fast diagnostic method to obtain denaturation profiles of biological samples using optical methods, that allows to diagnose various pathologies on a large scale and, possibly, to determine the degrees of evolution of said pathologies.

In accordance with a first aspect, the invention relates to a method for diagnosing a pathology in a mammal in need thereof, comprising the following steps:
using a biological sample collected from said mammal, said biological sample comprising a protein mixture, the biological sample being selected from a group consisting of blood, blood plasma and blood serum;
obtaining an optical thermal denaturation profile of said biological sample;
comparing said optical thermal denaturation profile of said biological sample with an optical thermal denaturation reference profile of mammals for which said pathology was diagnosed; and
diagnosing the pathology;
wherein the optical thermal denaturation profile is obtained by an optical method selected from the group consisting of fluorimetry, circular dichroism spectroscopy, infra-red spectroscopy and ultra-violet spectroscopy..

According to a second aspect, the invention relates to a system for diagnosing a pathology in a mammal in need thereof, said system comprising:
an apparatus for obtaining an optical thermal denaturation profile of a biological sample, said biological sample being selected from a group consisting of blood, blood plasma and blood serum, the optical thermal denaturation profile being obtained by an optical method selected from the group consisting of fluorimetry, circular dichroism spectroscopy, infra-red spectroscopy and ultra-violet spectroscopy;
a database comprising reference thermal denaturation profiles obtained from biological samples of healthy mammals and of mammals for which said pathology was diagnosed, the biological samples being selected from a group consisting of blood, blood plasma and blood serum; and
a computing system configured for comparing said optical thermal denaturation profile of said biological sample with the reference thermal denaturation profile, and diagnosing the pathology.

Advantageously, the method for diagnosing a pathology in a mammal in need thereof is characterized in that: - the biological sample is not diluted before obtaining the optical thermal denaturation profile of said biological sample; - the biological sample is depleted of at least one abundant protein of said biological sample, before obtaining the optical thermal denaturation profile of said biological sample; - the pathology is a brain disease, preferentially selected from the group consisting of neurodegenerative diseases and brain cancers; - the optical thermal denaturation profile is obtained by an optical method selected from the group consisting of differential scanning fluorimetry; - at least one fluorescent probe is added to the biological sample before obtaining an optical thermal denaturation profile of said biological sample; - the optical thermal denaturation profile is obtained for a first specific wavelength; - the optical thermal denaturation profile is obtained for a second specific wavelength; - the first and/or the second specific wavelength are approximately of 330 and/or 350 nm; - the optical thermal denaturation profile is a linear or non-linear combination of a plurality of optical thermal denaturation profiles obtained using various wavelength and/or various optical methods; - the method further comprising the step of providing a database, said database comprising optical thermal denaturation reference profiles; - the optical thermal denaturation reference profiles are obtained by: using biological samples collected from a plurality of healthy mammals and/or mammals for which a pathology was diagnosed; and obtaining optical thermal denaturation reference profiles from said biological samples; - the optical thermal denaturation profile obtained from the mammal in need thereof is compared with optical thermal denaturation reference profiles from healthy mammal and/or a mammal for which a pathology was diagnosed and the pathology is diagnosed when the optical thermal denaturation profile obtained from the mammal in need thereof differs from optical thermal denaturation reference profiles obtained from healthy mammals but matches optical thermal denaturation reference profiles obtained from mammals for which said pathology was diagnosed; - a degree of evolution of the pathology is deducted from a variation between the optical thermal denaturation profile obtained from the mammal in need thereof and the optical thermal denaturation reference profiles from mammals for which the pathology was diagnosed.

### BRIEF DESCRIPTION OF DRAWINGS

Other features and aspects of the present invention will be apparent from the following description and the accompanying drawings, in which:
Fig. 1 compares the denaturation profiles of a same human blood plasma sample obtained using DSC (grey curve) and DSF (black curve) according to the invention;
Fig. 2 provides average optical thermal denaturation profiles obtained using nanoDSF for several diseases according to the invention;
Fig. 3 provides an average of plasma optical-denaturation signatures obtained using nanoDSF for patients with glioblastoma;
Figs. 4A, 4B and 4C are panels that show the effect of dilution on the absorbance signals at 350 nm acquired for various plasma samples according to the invention;
Figs. 5A, 5B and 5C are panels that show the effect of dilution on the fluorescence ratio at 350 nm/330 nm acquired for various plasma samples according to the invention; and

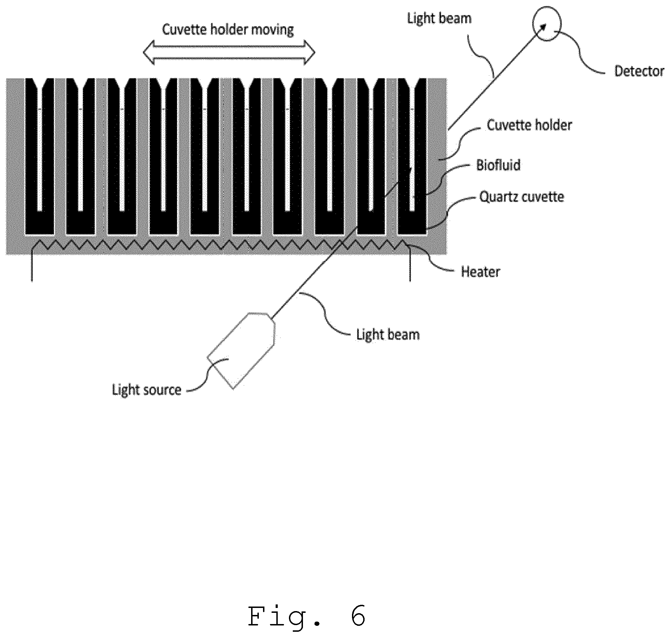

Fig. 6 represents an example of a typical multi-cuvette apparatus that allows recording of several optical thermal denaturation profiles of biological samples, simultaneously.

### DETAILLED DESCRIPTION OF THE INVENTION

The present invention relates to a method for diagnosing and/or following a pathology in a mammal in need thereof.

As used herein, the term "mammal" is intended to mean both human and non-human mammals. Nevertheless, the mammals, which are subject of this invention are, in particular, humans.

As used herein, the terms "biological sample" refer to biological fluids, biofluids or body fluids, that comprise at least a protein mixture, i.e. a mixture of a plurality of various proteins, for example a mixture of hundreds of proteins.

As used herein, the terms "optical thermal denaturation profile" are intended to mean a profile, or also called a thermogram, showing the denaturation of a biological sample, obtained by a method for evaluating optical properties of a solution as a function of temperature. Moreover, the "optical thermal denaturation profile" may also be assimilated to an "optical-denaturation signature" or "ODS".

As used herein, the term "approximately", when applied to a value X nm, means that said value is fixed at X nm, but may also range from X-10 nm to X+10 nm. Hence, the terms "approximately of 330 nm" mean that the wavelength is fixed at 330 nm, but may also range from 320 nm to 340 nm.

According to the invention, the method for diagnosing a pathology in a mammal in need thereof, comprises a first step of using a biological sample from said mammal.

The biological samples that may be used in the present invention include blood, blood plasma, blood serum, bone marrow, cerebral spinal fluid, urine, sweat or saliva. According to a preferred embodiment, the biological sample is selected from the group consisting of blood plasma and blood serum.

The biological samples comprise a protein mixture, generally a mixture of hundreds of proteins. For example, such a mixture is a mixture of all proteins that are usually contained in the blood plasma, if the biological sample is blood plasma. If so, it thus includes albumins, globulins, fibrinogens, regulatory proteins, clotting factors, etc. If the biological sample is blood serum, then it comprises the same proteins, except fibrinogens nor, possibly, clotting factors. The biological samples according to the invention however do not comprise only a mixture of proteins. It may comprises various molecules, including, for example, when the biological samples are blood plasma or serum, electrolytes such as Na⁺, Ca²⁺, Mg²⁺, HCO₃⁻ and Cl⁻, gases including carbon dioxide, or various nutrients such as glucose, amino-acids, fats, vitamins, minerals and lipids. Of course, the biological samples also contain water.

In some specific embodiments according to the present invention, a depletion, or an elimination, of at least one abundant protein that is contained in the biological sample may be carried out before obtaining an optical thermal denaturation profile of said biological sample. This depletion step avoids that the denaturation signals of abundant proteins in the sample mask signals of less abundant proteins. For instance, in some embodiments according to the invention, it may be advantageous to deplete a plasma sample of albumins or fibrinogens that constitute, approximately, 55% and 7% of the blood proteins, respectively. Indeed, the thermal denaturation profile of such abundant proteins is sometime not affected by a pathology. Removing some or all abundant proteins is generally a step that should be carried out according to the invention in order to evaluate if the less abundant proteins are affected by the disease. In certain cases, it is recorded a "second order" denaturation profile related to the denaturation of less abundant proteins in the biological sample. Indeed, the thermal denaturation profile related to less abundant proteins provides additional information.

The method according to the present invention further comprises a second step of obtaining an optical thermal denaturation profile of a biological sample. This profile is the first derivative of the temperature dependence of the fluorescence signal. The biological sample comprises at least a protein mixture that includes a plurality of various proteins wherein the most abundant provide the main contribution in such a profile. The sets of abundant proteins depend on the biofluid that is analyzed. For example, the human plasma contains notably albumin, immunoglobulin G (IGG), fibrinogen, immunoglobulin A, alpha-2-macroglobulin, haptoglobin and alpha-1-antitrypsin. Consequently, the denaturation of these proteins mainly impact plasma denaturation profile. Due to the biofluids homeostasis, the concentrations of these proteins are carefully maintained, which results in reproducible denaturation profile of plasma that does not depend on sex, race or age. However, in case of a disease, the denaturation profile of the biological sample changes due to modifications in concentration or thermostability of plasma abundant proteins. Such changes in proteins thermostability occur due to mutations, post-transitional modifications of the protein or its interaction with a biomarker, even if such a biomarker is not identified according to the invention. Generally, the thermal denaturation profile of a biological sample is also affected by interactions between proteins and other constituents of the biological sample, such as for example lipids.

The obtaining of the optical thermal denaturation profile is carried out advantageously without dilution of the biological sample. Indeed, as it will be demonstrated in the following example 4 according to the invention, dilution and, in particular dilution of 10-fold or more will lead to a lost a useful information for the implementation of the invention. However, a slight dilution, for example of less than 8-fold, preferentially from 2 to 8-fold, may bring some useful information for the implementation of the invention, in particular in connection with the nature of the proteins that form peaks that are lost/not lost with dilution. Thus, it may be useful to obtain an optical thermal denaturation profile of a non-diluted biological sample, and an additional optical thermal denaturation profile of the same biological sample, however slightly diluted, of less than 8-fold, preferentially from 2 to 8-fold, for example of a 5-fold dilution.

The optical thermal denaturation profiles according to the invention are advantageously obtained by an optical method selected from the group consisting of fluorimetry, circular dichroism (CD) spectroscopy, infra-red spectroscopy and ultra-violet spectroscopy. According to a preferred embodiment of the invention, the optical thermal denaturation profile of biological sample is obtained using a differential scanning fluorimetry method (DSF), including micro-DSF and nano-DSF.

Optical methods notably based on fluorimetry and CD spectroscopy have some peculiarities, namely the optical thermal denaturation profile of biological sample obtained using said optical methods for diagnostic purposes. DSF is a known method which monitors thermal unfolding of proteins via their intrinsic fluorescence or in the presence of additional fluorescence dye. DSF can be applied to a wide range of proteins. DSF is an excellent platform to screen for conditions that stabilize proteins. In DSF, the fluorescence intensity is plotted as a function of temperature, and this generates a sigmoidal curve that can be described by transition state. Typical denaturation profile is then obtained using first derivative of this curve.

DSF may be implemented using fluorescent probes or not.

When such probes are not used, fluorescence at a wavelength of approximately 350 nm is usually associated with thermostability of the tertiary structure of the proteins which contains a lot of aromatic amino acids, in particular tryptophan. More precisely, the fluorescence is associated with thermostability of local environment of tryptophan. So, if there is only one or a few tryptophans, the thermostability could be associated with thermostability of quaternary or secondary structure, depending on localization of this tryptophan. Thus, the optical thermal denaturation profile is associated with a set of proteins comprising a high amount of aromatic amino acids. On the other hand, the optical thermal denaturation profile obtained by CD spectroscopy at a wavelength of approximately 220 nm is associated with the thermal stability of secondary structure of the proteins with high amount of α-helix and β-sheets conformations. This allows to obtain complementary information using different optical methods and use it for diagnostic purposes.

According to another example, some fluorescent probes can be used for DSF. Such probes are, practically, fluorescent dyes. These fluorescent dyes are highly fluorescent in a non-polar environment, such as the hydrophobic sites on unfold proteins, as compared to aqueous solutions where the fluorescent is quenched. The various fluorescent dyes that have been used differ with respect to their optical properties, particularly in the fluorescence quantum yield caused by binding to denatured protein.

In another embodiment, at least one fluorescent probe is added to the biological sample before obtaining an optical thermal denaturation profile of said biological sample. It is noticed that any fluorescence probe known by the ordinary skill in the art can be used in the present invention. Among the fluorescent probes that can be used in the method of the present invention, it is herein disclosed 9-(diethylamino)-5H-Benzo[α]phenoxazin-5-one (Nile Red^{™}), SYPRO^{®} Orange Protein Gel Stain, 4-[5-[4-(dimethylamino)phenyl]-2-oxazolyl]-Benzenesulfonic acid sodium salt (Dapoxyl^{®} sulfonic acid sodium salt),4,4'-dianilino-1,1'-binaphthyl-5,5'-disulfonic acid dipotassium salt (bis-ANS^{™}), and 1-Anilinonaphthalene-8-Sulfonic Acid (1,8-ANS^{™}). The addition of at least one of these fluorescent probes allows firstly to follow proteins which are not naturally fluorescent and secondly to make the method of the present invention more sensitive for diagnosing a pathology.

In a specific embodiment of the invention, the optical thermal denaturation profile is obtained for a first specific wavelength (λ₁). In another specific embodiment, the optical thermal denaturation profile is obtained for a second specific wavelength (λ₂). The method for obtaining the optical thermal denaturation profile is advantageously measured at the first and/or at the second specific wavelength (λ₁, λ₂) which are approximately of 330 and/or 350 nm.

In a more preferred embodiment, the optical thermal denaturation profile is a linear or non-linear combination of a plurality of optical thermal denaturation profiles obtained using various wavelength and/or various optical methods. In other words, the evaluation of any optical property of biological sample as a function of temperature called here optical-denaturation signature (ODS) is useful for diagnostic purposes. This optical property could be a CD spectrum Θ(λ), a spectrum of fluorescence Φ(λ); a CD value at fixed wavelength Θ(λₙ); a fluorescence at a fixed wavelength **Φ** (λₙ); or any linear or non-linear combination of these properties, for example **Φ** (λₙ₁)/ **Φ** (λₙ₂). The temperature dependence of these properties may be described as a function of one or two variables, for example **Θ**λ₂₂₀ (T) or **Θ**(λ, T)). The function of two variables could be also reduced to the function of one variable in the following way **Θ**(Λ(T), T), where is λ=Λ (T).

The method according to the present invention further comprises a third step of comparing said optical thermal denaturation profile of said biological sample with an optical thermal denaturation reference profile. Such a comparison can be carried out by cluster analysis, machine learning or any other appropriate method. The optical thermal denaturation reference profile is extracted from a database, said database containing several optical denaturation profiles of the biological samples obtained firstly from healthy mammals and secondly from mammals for which a pathology was diagnosed. All the denaturation profiles in the database are used as reference profiles for a diagnosing purpose.

Hence, according to the present invention, the method for diagnosing a pathology in a mammal in need thereof involves said database comprising optical thermal denaturation reference profiles.

The optical thermal denaturation reference profiles are, in particular, obtained by:
using biological samples from a plurality of healthy mammals and/or mammals for which a pathology was diagnosed; and
obtaining optical thermal denaturation reference profiles from said biological samples.

Finally, the method according to the present invention further comprises a step of diagnosing the pathology. For that purpose, the optical thermal denaturation profile obtained from the mammal in need thereof is compared with the optical thermal denaturation reference profiles from healthy mammal and/or a mammal for which a pathology was diagnosed.

In preferred embodiment, the pathology is diagnosed when the optical thermal denaturation profile obtained from the mammal in need thereof differs from optical thermal denaturation reference profiles obtained from healthy mammals but matches optical thermal denaturation reference profiles obtained from mammals for which said pathology was diagnosed.

It is noted that the comparison between the optical thermal denaturation profile obtained from the mammal in need thereof and the optical thermal denaturation reference profiles obtained from healthy mammals, or the optical thermal denaturation reference profiles obtained from mammals for which said pathology was diagnosed, is carried out between the same type of mammals. Hence, the optical thermal denaturation profile obtained from a human in need thereof is compared to the optical thermal denaturation reference profiles obtained from healthy human.

A degree of evolution of a pathology may be deducted from a variation between the optical thermal denaturation profile obtained from a mammal and the optical thermal denaturation reference profiles from mammals for which the pathology was diagnosed.

The method according to the present invention may not lead to identifying a specific pathology, but to identifying if a denaturation profile is abnormal, i.e. to provide a diagnostic of an existing pathologic state for a mammal. Through the method of the present invention, an early diagnosing of a pathology is indeed possible by simply comparing the optical thermal denaturation reference profiles obtained from healthy mammals with the optical thermal denaturation profile obtained from the mammal in need thereof. Such a comparison allows to diagnose a general pathological state or a pathology which is at an early stage of development. In some cases, a pathology, which is at an early stage of development, does not involve clinical symptoms. When a disease is diagnosed at an advanced-stage, the treatment is not often a disease-modifying therapy but rather a treatment for decreasing the pathology symptoms. The method according to invention allows to early diagnose a pathology and also to measure the effect of a treatment with a view, for example, to provide some quick adaptation to such treatment, and determine which would be the most appropriate one.

The pathology, which is diagnosed according to the method of the invention, is, according to an embodiment, a brain disease. For example, such a disease is selected from the group consisting of the neurodegenerative diseases and the brain cancers. Brain cancers that can be diagnosed by the method of the present invention are, for example, glioblastomas, astrocystomas, oligodendrogliomas and ependymomas, pituitary adenomas, vestibular schwannomas and neuroectodermal tumors. Neurodegenerative diseases that can be diagnosed by the method of the present invention are, for example, Alzheimer's disease, Parkinson's disease, Huntington's disease, Prion disease, motor neuronal disease, spinocerebellar ataxia, spinal muscular atrophy, amyotrophic lateral sclerosis, Friedreich's ataxia, Lewy body disease.

Nevertheless, numerous pathologies may be diagnosed including, notably, diseases associated with pathological proteins aggregation, inflammatory diseases or autoimmune diseases.

It is noted that the diagnosis of brain diseases according to the state of the art may involve the step of taking a cerebral fluid sample. This is often difficult to practice and generally painful to the patient. Thanks to the method of the present invention, it is now possible to diagnose brain disease with a simple blood sample, in particular blood plasma or blood serum, which may appear surprising, as blood plasma and serum are separated from the brain tissue by blood brain barrier.

The present invention also relates to a system for diagnosing a pathology. This system comprises in particular an apparatus for obtaining an optical thermal denaturation profile of a biological sample. The system of the invention further comprises a database, said database including proteins reference thermal denaturation profiles obtained from biological sample of healthy mammals and/or of mammals for which said pathology was diagnosed. The system also comprises a computing system for comparing said optical thermal denaturation profile of said biological sample with the reference thermal denaturation profile, and then diagnosing the pathology.

Hence with the system of the present invention, it is possible to prevent or early diagnose a pathology. As above-mentioned in the description, when the disease is diagnosed at an advanced-stage, the treatment is not often a disease-modifying therapy but rather a treatment for decrease the side effects of the pathology. Hence with the system of the present invention, it is now possible to early diagnose a pathology and so adapt rapidly an appropriate treatment. It is also possible to diagnose a pathology and then adapt rapidly an appropriate treatment.

It is noted that the diagnosing of pathologies according to the state of the art usually involves the identification of specific biomarkers, such identification often requiring several years of pharmaceutical research. According to the method of the present invention, it is possible to diagnose a pathology in a mammal in need thereof without identifying the specific biomarkers of said pathology. The diagnosing of the pathology is both faster and easier to carry out.

### EXAMPLES

### 1. Denaturation profiles obtained using DSC and optical thermal denaturation profile using DSF.

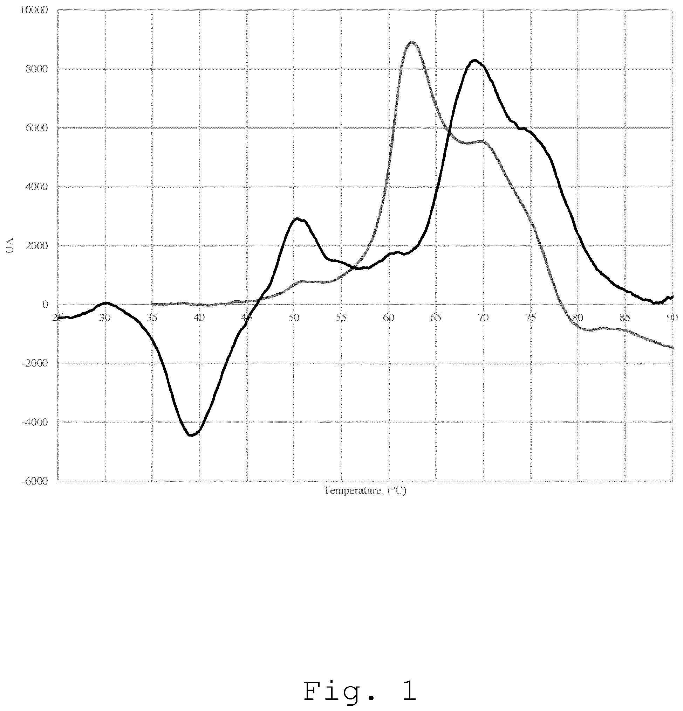

Fig. 1 shows two denaturation profiles of the same biological sample obtained using different methods. The grey curve represents a denaturation profile obtained by DSC, while the black one is obtained by nanoDSF. NanoDSF is an advanced Differential Scanning Fluorimetry method for measuring ultra-high resolution protein stability using intrinsic tryptophan or tyrosine fluorescence. The black curve is the first derivative of the temperature dependence of fluorescence signals ratio at 350 and 330 nm. It is noticed that the black curve has a pronounced negative peak in the region from 35°C to 45°C where the profile obtained using DSC is absolutely flat. Thus, the protein denaturation in this temperature interval impact only profiles obtained using nanoDSF but not DSC. So, if, in some diseases, the thermostability of this protein will be affected, only profiles obtained using nanoDSF could be used for diagnostic purposes. Moreover, the peaks at 50°C, 69°C shoulder at 75°C obtained by DSC, are less pronounced than in nanoDSF profile making nanoDSF more sensible to the changes associated with fibrinogen and IGG thermostability.

### 2. Optical thermal denaturation profile for diagnosing several diseases.

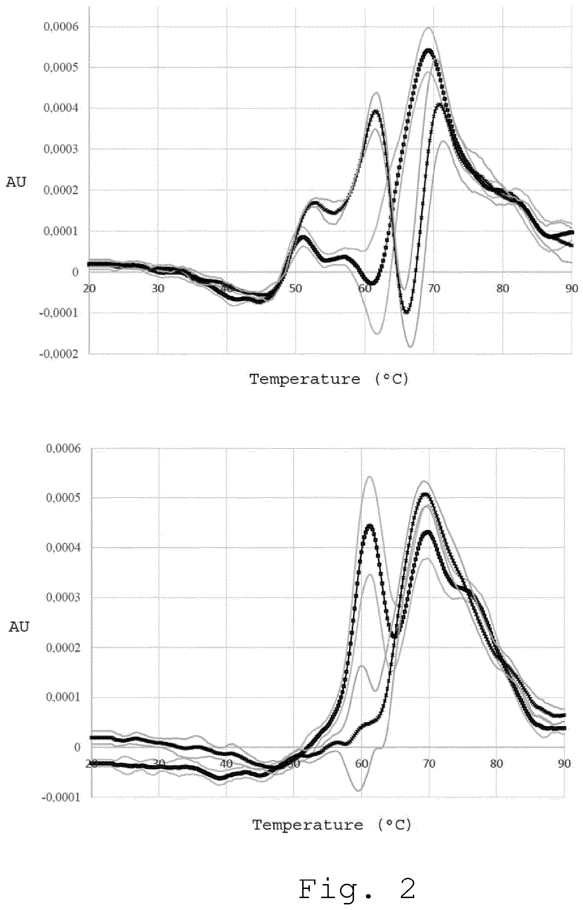

In Fig. 2 (top panel), two average plasma optical-denaturation signatures obtained using nanoDSF are shown. The black curve (square markers) represents the average from 8 plasma ODS from healthy individuals and the black curve (cross markers) represents the average from 5 ODS from Parkinson disease patients (the standard deviation is shown as grey/up and grey/low). Two average ODS, the black curve (square markers) and the black curve (cross markers), are clearly different from each other which makes them useful for potential diagnostic purposes. ODS of serum is also used for this purpose. In Fig. 2 (low panel), two average serum OSCs are shown. The black curve (square markers) represents the average from 12 serum ODSs from healthy individuals and the black curve (cross markers) represents the average from 10 ODS from patients that suffer from multiple sclerosis (the standard deviation is shown as grey/up and grey/low).

### 3. Optical thermal denaturation profile for diagnosing glioblastoma in patient in a need thereof.

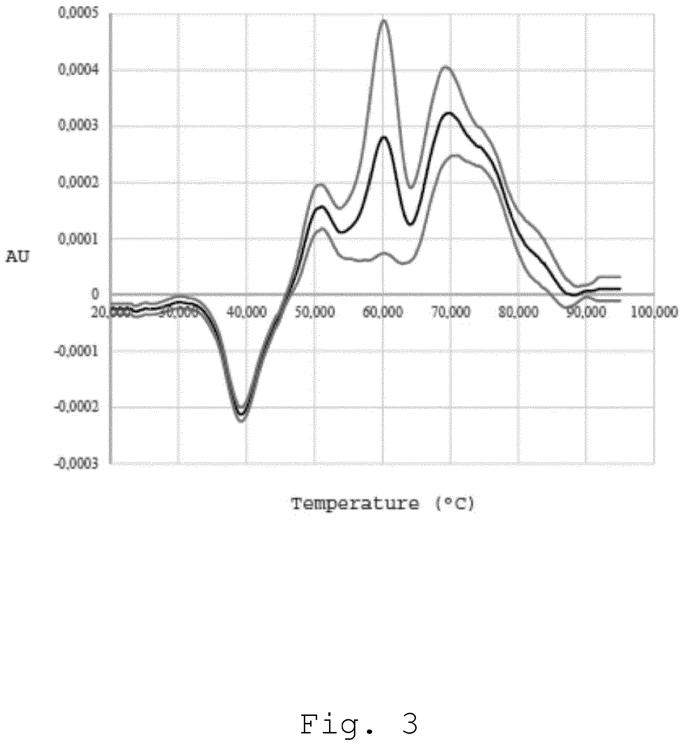

In Fig. 3, an average of plasma optical-denaturation signatures obtained using nanoDSF is shown. The black curve represents the average from 30 ODS from patients with glioblastoma (the standard deviation is shown as grey/up and grey/low).

### 4. Effect on the dilution of the biological sample

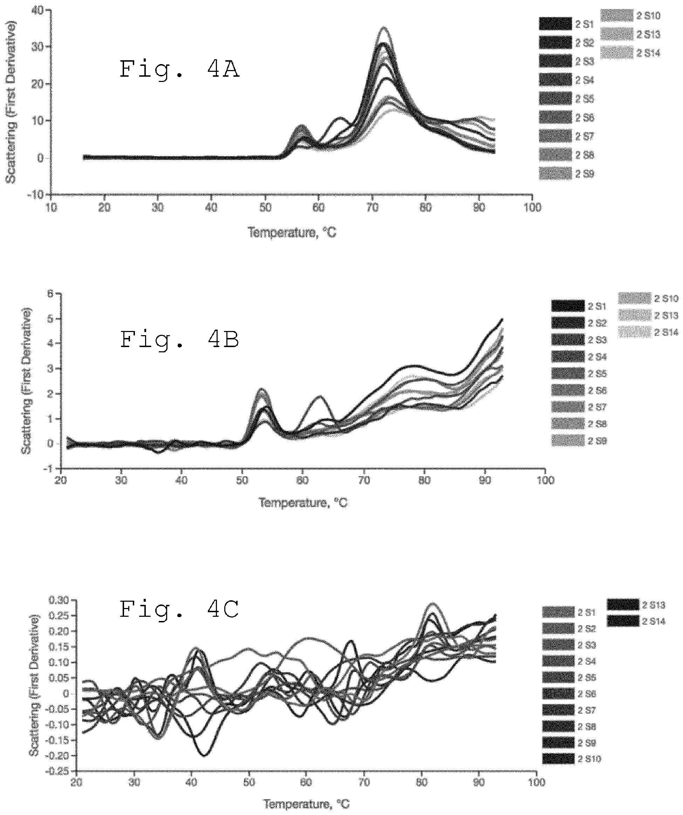

A first derivative of the absorbance at 350nm of plasma samples from 12 different mice, comprising a mix of control and mice grafted with different tumors was acquired according to the invention. The first panel in Fig. 4A corresponds to non-diluted plasma, the second panel in Fig. 4B to diluted plasma of 5-fold (1/5 dilution) and third panel in Fig. 4C to very diluted plasma of 20-fold (1/20 dilution).

As appearing on the figures 4A to 4C, the differences that can be seen in the first panel between the different mice samples are gradually lost with dilution. It is to be noted that, if the obvious conclusion from these figures is that, with dilution, specific signal is lost, it should be noted that if both not diluted and diluted tests are performed, information about the nature of the non-diluted peak is obtained. If it is lost with dilution, it probably means it was due to interaction with biomarkers - if it is not that may mean that the protein itself is modified.

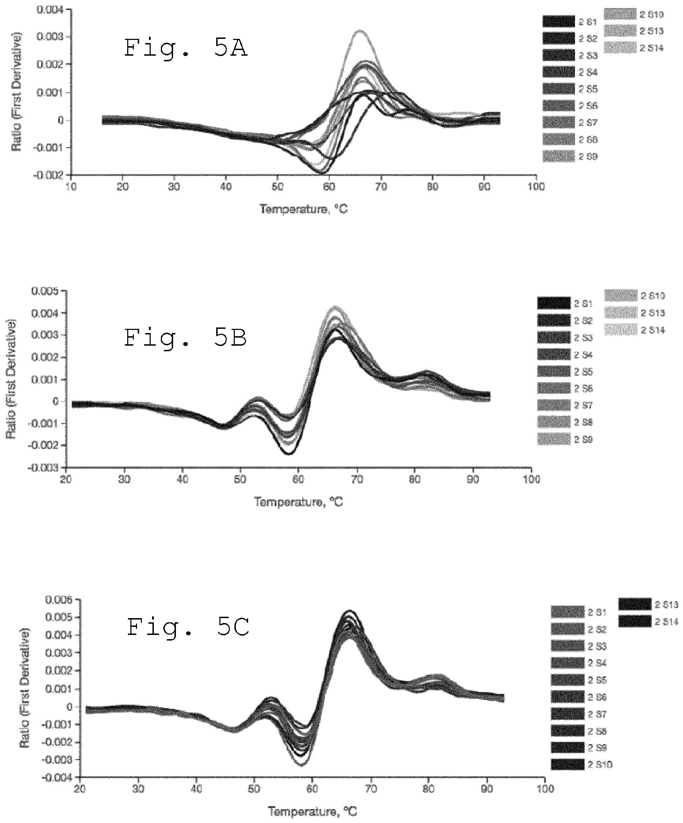

A first derivative of the fluorescence ratio (350 nm/ 330 nm) of plasma samples from 12 different mice, comprising a mix of control, and grafted mice with different tumors or treated mice, was acquired. The first panel in Fig. 5A corresponds to non-diluted plasma, the second panel in Fig. 5B to diluted plasma of 5-fold (1/5 dilution) and the third panel of Fig. 5C to very diluted plasma of 20-fold (1/20 dilution).

As appearing on the figures 5A to 5C, the differences that can be seen in the first panel between different mice are gradually lost with dilution. At a 20-fold dilution, we still have a signal but all the mice profiles are identical.

### 5. Optical method for screening high number of biological samples.

In order to obtain ODS of biological samples, any apparatus, which allow to register evaluation of some optical property of the solutions as a function of temperature, is suitable. Optical methods are in particular adopted to operate with a large number of biological samples. Indeed, since DSC apparatus register extremely low heat exchange, the experimental cells should be carefully isolated from external heat sources by adiabatic jackets. This makes highly difficult to create DSC apparatus that allow to run multiple samples at a same time. There are Multi Cell DSC apparatus, but their sensitivity is not enough to register denaturation profiles of biological samples. Moreover, sample cells are not replaceable and should be carefully washed after each sample. A number of spectroscopic and fluorimetry apparatus are able to read data from several removable cells simultaneously. For example, as shown in Fig. 4, a typical multi-cuvette apparatus that allows recording of several optical thermal denaturation profiles of biological samples comprises a cuvette holder. The cuvette holder comprises several single quartz cuvettes which contain biofluid samples. The multi-cuvette apparatus also comprises a heater which allows to heat the biofluid through a programmable temperature gradient. During all the heat experiment, a light source, typically an UV system, provides a light beam, which passes through the biofluid up to a detector. The detector is normally associated to a computing system for analyzing all the biological samples. Most suitable apparatus for working with large amount of biosamples available currently on the market is Prometheus NT.48^{™} from NanoTemper Technologies^{™}. It allows to run 48 samples at the same time and use disposable capillaries, which is very important when working with biofluids. While for DSC experiments, the biological samples have to be diluted 20-25 times in PBS, for nanoDSF experiments blood plasma is directly used without any pre-dilution step. Hence, Prometheus NT.48^{™} analyzes 48 samples in 30 minutes thanks to an improved heating rate (7°C/min with Prometheus NT.48^{™} *versus* 1.5°C/min with DSC). Prometheus NT.48^{™} enhances up to 200 time the analyzing speed in comparison to DSC method. Moreover, with Prometheus NT.48^{™}, capillaries are used instead of quartz cuvettes. Capillaries is more suitable than quartz cuvettes because capillaries are for a single use and it is not necessary to wash it after each experiment.

Hence, through optical thermal methods according to the invention, in particular DSF, it is now possible to analyze multiple biological samples at a same time. Also, using for example the specific apparatus described in Fig. 4, it is possible to develop a routine diagnostic approach which is faster than the DSC diagnostic method.

### 6. Conclusion

If due to changes in plasma homeostasis in some disease the most abundant proteins were not affected, denaturation signature obtained using DSC will not reveal any deviations. But, in the same time, if the protein with high amount of aromatic amino acids is affected then optical-denaturation signature obtained using fluorimetry is dramatically disturbed.

If, due to mutations or post-transitional modifications (such as phosphorylation etc.) in most abundant proteins, the thermostability of their tertiary structure is not affected but the thermostability of secondary structure is changed then the optical thermal denaturation profile obtained with CD spectroscopy at a wavelength approximately of 220nm (which follow the thermostability of secondary structure) is more informative than classical signature obtained by DSC. Thus, the optical thermal denaturation profile can reveal some disease-induced changes in the thermostability of plasma proteins, which cannot be detected by DSC.

## Claims

1. A method for diagnosing a pathology in a mammal in need thereof, comprising the following steps:
using a biological sample collected from said mammal, said biological sample comprising a protein mixture, the biological sample being selected from a group consisting of blood, blood plasma and blood serum;
obtaining an optical thermal denaturation profile of said biological sample;
comparing said optical thermal denaturation profile of said biological sample with an optical thermal denaturation reference profile of mammals for which said pathology was diagnosed; and
diagnosing the pathology;
wherein the optical thermal denaturation profile is obtained by an optical method selected from the group consisting of fluorimetry, circular dichroism spectroscopy, infra-red spectroscopy and ultra-violet spectroscopy.

2. The method according to claim 1, wherein the biological sample is not diluted before obtaining the optical thermal denaturation profile of said biological sample.

3. The method according to anyone of claims 1 or 2, wherein the biological sample is depleted of at least one abundant protein of said biological sample, before obtaining the optical thermal denaturation profile of said biological sample.

4. The method according to anyone of the preceding claims, wherein the pathology is a brain disease, preferentially selected from the group consisting of neurodegenerative diseases and brain cancers.

5. The method according to anyone of the preceding claims, wherein the optical thermal denaturation profile is obtained by an optical method selected from the group consisting of differential scanning fluorimetry.

6. The method according to anyone of the preceding claims, wherein at least one fluorescent probe is added to the biological sample before obtaining an optical thermal denaturation profile of said biological sample.

7. The method according to anyone of the previous claims, wherein the optical thermal denaturation profile is obtained for a first specific wavelength.

8. The method according to claim 7, wherein the optical thermal denaturation profile is obtained for a second specific wavelength.

9. The method according to anyone of the claims 7 or 8, wherein the first and/or the second specific wavelength are approximately of 330 and/or 350 nm.

10. The method according to anyone of the preceding claims, wherein the optical thermal denaturation profile is a linear or non-linear combination of a plurality of optical thermal denaturation profiles obtained using various wavelength and/or various optical methods.

11. The method according to anyone of the preceding claims, further comprising the step of providing a database, said database comprising optical thermal denaturation reference profiles.

12. The method according to claim 11, wherein the optical thermal denaturation reference profiles are obtained by:
using biological samples collected from a plurality of healthy mammals and/or mammals for which a pathology was diagnosed; and
obtaining optical thermal denaturation reference profiles from said biological samples.

13. The method according to anyone of the preceding claims, wherein the optical thermal denaturation profile obtained from the mammal in need thereof is compared with optical thermal denaturation reference profiles from healthy mammal and/or a mammal for which a pathology was diagnosed and the pathology is diagnosed when the optical thermal denaturation profile obtained from the mammal in need thereof differs from optical thermal denaturation reference profiles obtained from healthy mammals but matches optical thermal denaturation reference profiles obtained from mammals for which said pathology was diagnosed.

14. The method according to claim 13, wherein a degree of evolution of the pathology is deducted from a variation between the optical thermal denaturation profile obtained from the mammal in need thereof and the optical thermal denaturation reference profiles from mammals for which the pathology was diagnosed.

15. A system for diagnosing a pathology in a mammal in need thereof, said system comprising:
an apparatus for obtaining an optical thermal denaturation profile of a biological sample, said biological sample being selected from a group consisting of blood, blood plasma and blood serum, the optical thermal denaturation profile being obtained by an optical method selected from the group consisting of fluorimetry, circular dichroism spectroscopy, infra-red spectroscopy and ultra-violet spectroscopy;
a database comprising reference thermal denaturation profiles obtained from biological samples of healthy mammals and of mammals for which said pathology was diagnosed, the biological samples being selected from a group consisting of blood, blood plasma and blood serum; and
a computing system configured for comparing said optical thermal denaturation profile of said biological sample with the reference thermal denaturation profile, and diagnosing the pathology.

## Patentansprüche

1. Verfahren zur Diagnose einer Pathologie bei einem Säugetier, das einer solchen Diagnose bedarf, umfassend die folgenden Schritte:
Verwenden einer biologischen Probe, die von dem Säugetier entnommen wurde, wobei die biologische Probe eine Proteinmischung umfasst und aus einer Gruppe ausgewählt ist, die aus Blut, Blutplasma und Blutserum besteht;
Erhalten eines optischen thermischen Denaturierungsprofils der biologischen Probe;
Vergleichen des optischen thermischen Denaturierungsprofils der biologischen Probe mit einem optischen thermischen Denaturierungsreferenzprofil von Säugetieren, bei denen diese Pathologie diagnostiziert wurde; und
Diagnostizieren der Pathologie;
wobei das optische thermische Denaturierungsprofil durch ein optisches Verfahren erhalten wird, das aus der Gruppe ausgewählt ist, die aus Fluorimetrie, Zirkulardichroismusspektroskopie, Infrarotspektroskopie und Ultraviolettspektroskopie besteht.

2. Verfahren nach Anspruch 1, wobei die biologische Probe vor dem Erhalten des optischen thermischen Denaturierungsprofils dieser biologischen Probe nicht verdünnt wird.

3. Verfahren nach einem der Ansprüche 1 oder 2, wobei die biologische Probe an mindestens einem darin reichlich vorhandenen Protein abgereichert wird, bevor das optische thermische Denaturierungsprofil der biologischen Probe erhalten wird.

4. Verfahren nach einem der vorstehenden Ansprüche, wobei die Pathologie eine Hirnerkrankung ist, die vorzugsweise aus der Gruppe ausgewählt ist, die aus neurodegenerativen Erkrankungen und malignen Hirntumoren besteht.

5. Verfahren nach einem der vorstehenden Ansprüche, wobei das optische thermische Denaturierungsprofil durch ein optisches Verfahren erhalten wird, das aus der Gruppe ausgewählt ist, die aus der Differential Scanning-Fluorimetrie besteht.

6. Verfahren nach einem der vorstehenden Ansprüche, wobei der biologischen Probe mindestens eine Fluoreszenzsonde zugesetzt wird, bevor ein optisches thermisches Denaturierungsprofil dieser biologischen Probe erhalten wird.

7. Verfahren nach einem der vorstehenden Ansprüche, wobei das optische thermische Denaturierungsprofil für eine erste spezifische Wellenlänge erhalten wird.

8. Verfahren nach Anspruch 7, wobei das optische thermische Denaturierungsprofil für eine zweite spezifische Wellenlänge erhalten wird.

9. Verfahren nach einem der Ansprüche 7 oder 8, wobei die erste und/oder die zweite spezifische Wellenlänge etwa 330 und/oder 350 nm beträgt.

10. Verfahren nach einem der vorstehenden Ansprüche, wobei das optische thermische Denaturierungsprofil eine lineare oder nichtlineare Kombination aus einer Vielzahl von optischen thermischen Denaturierungsprofilen ist, die unter Verwendung verschiedener Wellenlängen und/oder verschiedener optischer Verfahren erhalten wurden.

11. Verfahren nach einem der vorstehenden Ansprüche, ferner umfassend den Schritt des Bereitstellens einer Datenbank, wobei die Datenbank optische thermische Denaturierungsreferenzprofile umfasst.

12. Verfahren nach Anspruch 11, wobei die optischen thermischen Denaturierungsreferenzprofile wie folgt erhalten werden:
Verwenden biologischer Proben, die von einer Vielzahl gesunder Säugetiere und/oder von Säugetieren entnommen wurden, bei denen eine Pathologie diagnostiziert wurde; entnommen wurden und
Erhalten optischer thermischer Denaturierungsreferenzprofile aus den biologischen Proben.

13. Verfahren nach einem der vorstehenden Ansprüche, wobei das von dem betreffenden Säugetier erhaltene optische thermische Denaturierungsprofil mit optischen thermischen Denaturierungsreferenzprofilen von gesunden Säugetieren und/oder von Säugetieren, bei denen eine Pathologie diagnostiziert wurde, verglichen wird, und die Pathologie diagnostiziert wird, wenn das von dem betreffenden Säugetier erhaltene optische thermische Denaturierungsprofil von den optischen thermischen Denaturierungsreferenzprofilen gesunder Säugetiere abweicht, jedoch mit den optischen thermischen Denaturierungsreferenzprofilen von Säugetieren übereinstimmt, bei denen die Pathologie diagnostiziert wurde.

14. Verfahren nach Anspruch 13, wobei der Grad des Fortschreitens der Pathologie aus der Abweichung zwischen dem optischen thermischen Denaturierungsprofil, das bei dem betreffenden Säugetier erhalten wurde, und den optischen thermischen Denaturierungsreferenzprofilen von Säugetieren, bei denen die Pathologie diagnostiziert wurde, abgeleitet wird.

15. System zur Diagnose einer Pathologie bei einem Säugetier, das einer solchen Diagnose bedarf, das System umfassend:
ein Gerät zum Erhalten eines optischen thermischen Denaturierungsprofils einer biologischen Probe, wobei die biologische Probe aus einer Gruppe ausgewählt ist, die aus Blut, Blutplasma und Blutserum besteht, und wobei das optische thermische Denaturierungsprofil durch ein optisches Verfahren erhalten wird, das aus der Gruppe ausgewählt ist, die aus Fluorimetrie, Zirkulardichroismusspektroskopie, Infrarotspektroskopie und Ultraviolettspektroskopie besteht;
eine Datenbank, umfassend thermische Denaturierungsreferenzprofile, die aus biologischen Proben gesunder Säugetiere sowie aus biologischen Proben von Säugetieren erhalten wurden, bei denen die Pathologie diagnostiziert wurde, wobei die biologischen Proben aus einer Gruppe ausgewählt sind, die aus Blut, Blutplasma und Blutserum besteht; und
ein Rechensystem, das dazu konfiguriert ist, das optische thermische Denaturierungsprofil der biologischen Probe mit dem thermischen Denaturierungsreferenzprofil zu vergleichen und die Pathologie zu diagnostizieren.

## Revendications

1. Procédé permettant de diagnostiquer une pathologie chez un mammifère qui en a besoin, comprenant les étapes suivantes :
l'utilisation d'un échantillon biologique prélevé sur ledit mammifère, ledit échantillon biologique comprenant un mélange de protéines, l'échantillon biologique étant choisi dans un groupe constitué de sang, de plasma sanguin et de sérum sanguin ;
l'obtention d'un profil de dénaturation thermique optique dudit échantillon biologique ;
la comparaison dudit profil de dénaturation thermique optique dudit échantillon biologique avec un profil de dénaturation thermique optique de référence des mammifères chez lesquels ladite pathologie a été diagnostiquée ; et
le diagnostic de la pathologie ;
dans lequel le profil de dénaturation thermique optique est obtenu par un procédé optique choisi dans le groupe constitué de fluorimétrie, de spectroscopie de dichroïsme circulaire, de spectroscopie infrarouge et de spectroscopie ultraviolette.

2. Procédé selon la revendication 1, dans lequel l'échantillon biologique n'est pas dilué avant d'obtenir le profil de dénaturation thermique optique dudit échantillon biologique.

3. Procédé selon l'une quelconque des revendications 1 ou 2, dans lequel l'échantillon biologique est appauvri en au moins une protéine abondante dudit échantillon biologique, avant d'obtenir le profil de dénaturation thermique optique dudit échantillon biologique.

4. Procédé selon l'une quelconque des revendications précédentes, dans lequel la pathologie est une maladie cérébrale, préférentiellement choisie dans le groupe constitué des maladies neurodégénératives et des cancers du cerveau.

5. Procédé selon l'une quelconque des revendications précédentes, dans lequel le profil de dénaturation thermique optique est obtenu par un procédé optique choisi dans le groupe constitué de fluorimétrie à balayage différentiel.

6. Procédé selon l'une quelconque des revendications précédentes, dans lequel au moins une sonde fluorescente est ajoutée à l'échantillon biologique avant d'obtenir un profil de dénaturation thermique optique dudit échantillon biologique.

7. Procédé selon l'une quelconque des revendications précédentes, dans lequel le profil de dénaturation thermique optique est obtenu pour une première longueur d'onde spécifique.

8. Procédé selon la revendication 7, dans lequel le profil de dénaturation thermique optique est obtenu pour une seconde longueur d'onde spécifique.

9. Procédé selon l'une quelconque des revendications 7 ou 8, dans lequel la première et/ou la seconde longueur d'onde spécifique sont approximativement de 330 et/ou 350 nm.

10. Procédé selon l'une quelconque des revendications précédentes, dans lequel le profil de dénaturation thermique optique est une combinaison linéaire ou non linéaire d'une pluralité de profils de dénaturation thermique optique obtenus en utilisant diverses longueurs d'onde et/ou de divers procédés optiques.

11. Procédé selon l'une quelconque des revendications précédentes, comprenant en outre l'étape consistant à fournir une base de données, ladite base de données comprenant des profils de dénaturation thermique optique de référence.

12. Procédé selon la revendication 11, dans lequel les profils de dénaturation thermique optique de référence sont obtenus par :
l'utilisation d'échantillons biologiques prélevés sur une pluralité de mammifères sains et/ou de mammifères chez lesquels une pathologie a été diagnostiquée ; et
l'obtention de profils de dénaturation thermique optique de référence à partir desdits échantillons biologiques.

13. Procédé selon l'une quelconque des revendications précédentes, dans lequel le profil de dénaturation thermique optique obtenu à partir du mammifère qui en a besoin est comparé à des profils de dénaturation thermique optique de référence provenant de mammifère sain et/ou d'un mammifère chez lequel une pathologie a été diagnostiquée et la pathologie est diagnostiquée lorsque le profil de dénaturation thermique optique obtenu à partir du mammifère qui en a besoin diffère des profils de dénaturation thermique optique de référence obtenus à partir de mammifères sains mais correspond à des profils de dénaturation thermique optique de référence obtenus à partir de mammifères chez lesquels ladite pathologie a été diagnostiquée.

14. Procédé selon la revendication 13, dans lequel un degré d'évolution de la pathologie est déduit d'une variation entre le profil de dénaturation thermique optique obtenu à partir du mammifère qui en a besoin et les profils de dénaturation thermique optique de référence à partir de mammifères chez lesquels la pathologie a été diagnostiquée.

15. Système permettant de diagnostiquer une pathologie chez un mammifère qui en a besoin, ledit système comprenant :
un appareil permettant d'obtenir un profil de dénaturation thermique optique d'un échantillon biologique, ledit échantillon biologique étant choisi dans un groupe constitué de sang, de plasma sanguin et de sérum sanguin, le profil de dénaturation thermique optique étant obtenu par un procédé optique choisi dans le groupe constitué de fluorimétrie, de spectroscopie de dichroïsme circulaire, de spectroscopie infrarouge et de spectroscopie ultraviolette ;
une base de données comprenant des profils de dénaturation thermique de référence obtenus à partir d'échantillons biologiques de mammifères sains et de mammifères chez lesquels ladite pathologie a été diagnostiquée, les échantillons biologiques étant choisis dans un groupe constitué de sang, de plasma sanguin et de sérum sanguin ; et
un système informatique configuré pour comparer ledit profil de dénaturation thermique optique dudit échantillon biologique avec le profil de dénaturation thermique de référence, et diagnostiquer la pathologie.
